# FASCICULE DE BREVET EUROPEEN

(11) **EP 3 465 184 B1**
(45) Date de publication et mention de la délivrance du brevet: **10.01.2024**
(21) Numéro de dépôt: 17729427.9
(22) Date de dépôt: 02.06.2017
(51) Int. Cl.: G01N 23/223, G01N 33/28, G01N 33/30

(54) **INSTALLATION ET PROCEDE DE SUIVI DE L'EVOLUTION DE LA QUALITE D'UN LUBRIFIANT ET METHODE DE SUIVI POUR LA DETERMINATION DE LA TENEUR EN FER D'UN LUBRIFIANT**
ANLAGE UND VERFAHREN ZUR ÜBERWACHUNG DER ÄNDERUNG DER QUALITÄT EINES SCHMIERMITTELS UND VERFAHREN ZUR BESTIMMUNG DES EISENGEHALTES EINES SCHMIERMITTELS
FACILITY AND METHOD FOR MONITORING THE CHANGE IN QUALITY OF A LUBRICANT AND MONITORING METHOD FOR DETERMINING THE IRON CONTENT OF A LUBRICANT

(30) Priorité: 02.06.2016 FR 1655022
(43) Date de publication de la demande: 10.04.2019
(73) Titulaire: TotalEnergies OneTech, 92400 Courbevoie (FR)
(72) Inventeur: JUSTON, Raphael, 73000 Chamberry (FR); TROADEC, Yann, 38000 Grenoble (FR); CHAUDOREILLE, François, 73100 Gresy Sur Aix (FR); ROMAN, Jean-Philippe, 38780 Septeme (FR); AMIOT, Arnaud, 92500 Rueil Malmaison (FR)
(74) Mandataire: Lavoix
(86) Numéro de dépôt international: PCT/EP2017/063427
(87) Numéro de publication internationale: WO 2017/207747

(56) Documents cités:
- AU-A1- 2005 201 261
- CN-A- 103 398 923
- US-A- 5 982 847
- US-A1- 2002 149 768
- US-A1- 2004 008 816
- US-B1- 7 016 462
- SHARMA M ET AL: "An EDXRF study of the photodecomposition of diorganyl ditellurides", RADIATION PHYSICS AND CHEMISTRY, ELSEVIER, AMSTERDAM, NL, vol. 75, no. 11, 10 juillet 2006 (2006-07-10), pages 2029-2038, XP027963269, ISSN: 0969-806X [extrait le 2006-11-01]

## Description

La présente invention a trait à une installation de suivi de l'évolution de la qualité d'un lubrifiant circulant dans un équipement, tel qu'un moteur de navire. L'invention a également trait à un procédé de suivi de l'évolution de la teneur en un élément chimique prédéterminé d'un lubrifiant, notamment en fer dissout et/ou particulaire d'un lubrifiant. En outre, l'invention a trait à une méthode de suivi du fonctionnement d'un équipement embarqué sur un navire.

Le domaine de l'invention est celui des équipements d'analyse des lubrifiants, notamment pour moteurs, en particulier pour les moteurs marins.

Dans le domaine des moteurs à combustion interne utilisés sur les navires de commerce, il est connu qu'il convient de suivre la situation d'un moteur en analysant le lubrifiant circulant dans ce moteur. Une telle analyse permet de détecter des phénomènes d'usure et/ou de corrosion qui ont tendance à se produire dans un moteur. Par le passé, le fonctionnement des moteurs était relativement stabilisé et il suffisait de contrôler la qualité d'un lubrifiant de façon ponctuelle, lors des escales, pour anticiper les opérations de maintenance à réaliser. De nos jours, les moteurs sont de plus en plus élaborés et sensibles aux phénomènes d'usure et/ou de corrosion, de sorte que des analyses doivent être réalisées à bord des navires et en mer, notamment pour suivre la teneur globale en fer du lubrifiant, également appelé huile ou huile moteur, cette teneur résultant de phénomènes d'abrasion. La teneur globale en fer du lubrifiant comprend la teneur en fer présent dans le lubrifiant sous la forme particulaire, par exemple en tant qu'oxyde de fer, et sous la forme dissoute, par exemple sous la forme ionique. Ceci impose de former le personnel et d'embarquer un matériel élaboré, dont le fonctionnement est relativement difficile à maitriser, même par le personnel naviguant formé. En outre, ceci augmente la charge de travail du personnel.

WO-A-2010/046591 prévoit d'utiliser un système embarqué dans lequel l'huile sortant d'un moteur est dirigée vers un composant fonctionnel associé à un système de mesure permettant de déterminer son indice de basicité ou sa teneur en particules métalliques. En pratique, le débit d'huile sortant du moteur est faible et l'écoulement en sortie du moteur est constitué de gouttelettes qui ruissèlent à l'intérieur d'une canalisation, au point qu'il n'est pas certain que le composant fonctionnel soit alimenté avec un débit d'huile suffisant pour que les mesures qu'il effectue soient correctes.

WO-A-03/091550 divulgue une méthode d'analyse d'un lubrifiant dans laquelle une mesure, effectuée au moyen d'un capteur XRF sur un échantillon d'un lubrifiant à contrôler, est comparée à des mesures effectuées sur des échantillons de lubrifiant de référence. Cette démarche est prévue pour un fonctionnement en laboratoire et requiert une main d'oeuvre qualifiée.

Il est par ailleurs connu de US-A-5 982 847 d'utiliser un spectromètre comprenant une source de rayons X et un détecteur de rayons X pour mesurer la teneur en particules métalliques dans un lubrifiant dues à l'usure du système de lubrification d'un moteur. Cette source et ce détecteur de rayons X sont associés à une cellule réalisée dans un matériau non métallique ou à base d'aluminium et qui est équipée d'une fenêtre transparente aux rayons X et qui peut être réalisée, par exemple, en béryllium, en bore ou en polymère revêtu d'aluminium. Le béryllium donne des résultats satisfaisants mais peut s'avérer toxique pour un opérateur. Les autres matériaux ont tendance à filtrer les rayons X. Compte tenu de la pression et de la température du fluide circulant dans la cellule, la fenêtre doit être relativement épaisse, pour résister aux efforts mécaniques qu'elle subit mais ceci a pour effet d'atténuer le rayonnement qui la traverse, à la fois entre la source de rayon X et l'échantillon à analyser et entre cet échantillon et le détecteur de rayons.

D'autre part, les équipements de laboratoires, tel que celui connu de US-B-6 233 307 qui pourrait permettre de détecter la teneur en fer dissout dans un lubrifiant, sont difficilement transportables et d'une utilisation complexe, ce qui les rend peu accessibles au personnel naviguant, même formé pour cela.

Il est également connu de AU-A-2005201261 de monter un système multi-capteur entre une ligne de circulation d'huile et un bac de récupération, ce système comprenant, entre autres, un réservoir de refroidissement disposé en aval d'une vanne principale et d'une vanne de calibration et en amont d'un capteur à fluorescence X comprenant un tube dans lequel s'écoule l'huile à analyser et qui est, de préférence, réalisé en polyamide et doit résister aux vibrations, tout en étant peu épais. Le polyamide risque d'être altéré par l'huile et de perturber la mesure effectuée à travers le tube.

Ces problèmes se posent non seulement sur les moteurs deux temps ou quatre temps de propulsion des navires, mais également sur d'autres moteurs secondaires également embarqués sur des navires, par exemple pour des accessoires de type palan. De façon générale, la surveillance de la teneur globale en fer, c'est-à-dire la teneur en fer dissout et/ou particulaire, dans un lubrifiant est importante pour tous les moteurs lubrifiés et les techniques connues sont peu propices à une automatisation.

Des problèmes analogues se posent pour la détermination de la teneur d'un lubrifiant en un autre élément chimique prédéterminé, notamment en calcium, vanadium, chrome, molybdène, cuivre, soufre, plomb, argent, étain, aluminium, nickel, zinc ou en phosphore.

C'est à ces inconvénients qu'entend plus particulièrement remédier l'invention en proposant une nouvelle installation de suivi de l'évolution de la qualité d'un lubrifiant circulant dans un équipement qui est adapté pour fonctionner de façon simple et autonome, ce qui libère notamment le personnel embarqué à bord d'un navire de tâches répétitives et élaborées.

A cet effet, l'invention concerne une installation de suivi de l'évolution de la qualité d'un lubrifiant circulant dans un équipement, cette installation comprenant au moins une conduite de circulation du lubrifiant, cette conduite étant raccordée, en amont, à l'équipement et, en aval, à un bac de récupération. Cette installation comprend, en outre, un capteur de détermination, par la technologie de fluorescence X, de la teneur globale en un élément chimique prédéterminé, d'un échantillon de lubrifiant, ce capteur comprenant une source de rayons X, un détecteur de rayons X et une cellule destinée à contenir un échantillon de lubrifiant à analyser et comprenant un boîtier. L'installation comprend également un réservoir. Conformément à l'invention, le réservoir est un réservoir tampon d'accumulation du lubrifiant. En outre, cette installation comprend une première vanne d'interruption commandée de la circulation du lubrifiant dans la conduite, une première ligne de dérivation raccordée d'une part à la conduite, en amont de la première vanne et d'autre part au réservoir tampon, une deuxième vanne d'interruption commandée de la circulation du lubrifiant dans la première ligne de dérivation, une deuxième ligne d'évacuation du lubrifiant, du réservoir tampon vers le bac de récupération, ainsi qu'une troisième vanne d'interruption commandée de la circulation du lubrifiant dans la deuxième ligne d'évacuation. Le capteur détermine la teneur globale en un élément chimique prédéterminé d'un échantillon de lubrifiant en sortie du réservoir tampon. En outre, le boîtier de la cellule est en métal et une paroi, qui forme une fenêtre de passage des rayons X issus de la source ou allant vers le détecteur, est rapportée sur le boîtier de la cellule et cette paroi est réalisée en poly(téréphtalate d'éthylène).

Grâce à l'invention, le capteur qui fonctionne par la technologie de fluorescence X permet une mesure embarquée de la teneur globale en un élément chimique prédéterminé dans un lubrifiant. Au sens de la présente invention, la teneur globale en un élément chimique dans un lubrifiant est la teneur en élément dissout et particulaire de cet élément chimique dans ce lubrifiant. De façon avantageuse, le capteur est un capteur de la teneur en fer dans le lubrifiant. La mesure réalisée n'est pas affectée, de façon sensible, par la paroi qui constitue la fenêtre de passage des rayons X. En effet, cette paroi, réalisée en poly(téréphtalate d'éthylène) ou PET, peut être prévue avec une épaisseur relativement peu importante, notamment inférieure à 200 µm (micromètre), tout en présentant des propriétés mécaniques satisfaisantes pour résister aux efforts de pression et de vibrations au sein d'une installation conforme à l'invention. Le matériau constitutif de la paroi lui permet également de ne pas être altéré par le lubrifiant qui circule dans la cellule.

Le lubrifiant dont il est question dans la présente invention est un lubrifiant classiquement utilisé et connu par l'homme du métier et qui permet de lubrifier un équipement, notamment un moteur de navire. Ce lubrifiant comprend au moins une huile de base lubrifiante. En général, les huiles de base lubrifiantes peuvent être des huiles d'origine minérales, synthétiques ou végétales ainsi que leurs mélanges.

Dans les lubrifiants, les huiles de base lubrifiantes peuvent être utilisées seules ou en mélange. Par exemple, une huile minérale peut être combinée avec une huile synthétique. Le lubrifiant peut comprendre, en outre, au moins un additif classiquement utilisé et connu par l'homme du métier pour la formulation de lubrifiant et plus particulièrement de lubrifiant marin. A titre d'exemple, l'additif peut être choisi parmi les détergents surbasés, les détergents neutres, les dispersants, les additifs anti-usure, tout autre additif fonctionnel, ou leurs mélanges.

Par exemple, le détergent surbasé et le détergent neutre peuvent être choisis parmi les carboxylates, sulfonates, salicylates, naphténates, phénates, et les détergents mixtes associant au moins deux de ces types de détergents. Le détergent surbasé et le détergent neutre sont notamment des composés à base de métaux choisis parmi le calcium, le magnésium, le sodium ou le baryum, préférentiellement le calcium ou le magnésium. Le détergent surbasé peut être surbasé par des sels insolubles métalliques choisis dans le groupe des carbonates de métaux alcalins et alcalino-terreux, préférentiellement le carbonate de calcium. Le lubrifiant peut comprendre au moins un détergent surbasé et au moins un détergent neutre tels que définis ci-dessus.

Par exemple, les composés dérivés de l'acide succinique sont des dispersants particulièrement utilisés comme additifs de lubrification. Les bases de Mannich, obtenues par polycondensation de phénols substitués par des groupements alkyles, de formaldéhyde et d'amines primaires ou secondaires, sont également des composés utilisés comme dispersants dans les lubrifiants.

A titre d'exemple, parmi les additifs anti-usure classiquement utilisé pour formuler les lubrifiants marins, l'additif anti-usure le plus couramment utilisé est le di thiophosphate de zinc ou DTPZn.

Par exemple, les autres additifs fonctionnels peuvent être choisis parmi les agents épaississants, les additifs anti-mousse pour contrer l'effet des détergents, pouvant être par exemple des polymères polaires tels que polyméthylsiloxanes, polyacrylates, les additifs anti-oxydant et/ou anti rouille, par exemple détergents organo-métalliques ou thiadiazoles.

Selon des aspects avantageux mais non obligatoires, une installation conforme à l'invention peut incorporer une ou plusieurs des caractéristiques suivantes, prises dans toute combinaison techniquement admissible :
- La cellule comprend un logement creux de réception de la paroi et une rondelle filetée d'immobilisation de la paroi dans le logement creux.
- Le capteur est un capteur de détermination de la teneur en fer de l'échantillon de lubrifiant.
- Le boîtier est réalisé dans un alliage non ferreux, notamment dans un alliage à base d'aluminium.
- La source de rayons X et le détecteur de rayon X sont montés sur un capot qui les positionne par rapport au boîtier et à la paroi, alors que ce support forme un blindage contre la propagation des rayons X.
- Un axe de visée de la source de rayons X et un axe de visée du détecteur de rayons X forment entre eux un angle compris entre 20° et 25°, de préférence de l'ordre de 22°.
- La paroi a une épaisseur inférieure ou égale à 200 µm, de préférence inférieure ou égale à 150 µm, de préférence encore de l'ordre de 125 µm.
- Le capteur de détermination de la teneur en un élément chimique prédéterminé est disposé sur la deuxième ligne d'évacuation
- L'installation comprend, en outre, un capteur de détermination de l'indice de basicité du lubrifiant, disposé sur la deuxième ligne d'évacuation et qui permet de déterminer l'indice de basicité du lubrifiant en sortie du réservoir tampon.

Par ailleurs, l'invention concerne un procédé automatisé de suivi de l'évolution de la teneur globale en un élément chimique prédéterminé dans un lubrifiant circulant dans un équipement, au moyen d'une installation telle que mentionnée ci-dessus, dans laquelle le capteur de détermination de la teneur en fer est disposé sur la deuxième ligne d'évacuation. Ce procédé est caractérisé en ce qu'il comprend au moins des étapes consistant à :
a) fermer la première vanne,
b) ouvrir la deuxième vanne et fermer la troisième vanne pour alimenter le réservoir tampon à partir d'une quantité de lubrifiant accumulée dans la conduite, en amont de la première vanne,
c) ouvrir la troisième vanne pour faire circuler le lubrifiant présent dans le réservoir tampon à travers la deuxième ligne d'évacuation jusque dans une cellule du capteur,
d) utiliser le capteur pour déterminer la teneur globale en un élément chimique prédéterminé du lubrifiant en sortie du réservoir tampon.

L'invention concerne également une méthode de suivi du fonctionnement d'un équipement embarqué sur un navire, cette méthode comprenant la détermination, à bord du navire, de la teneur globale en un élément chimique prédéterminé, notamment en fer, du lubrifiant circulant dans l'équipement en question par la mise en oeuvre d'un procédé automatisé tel que mentionné ci-dessus.

Enfin, l'invention concerne l'utilisation d'une méthode automatisée telle que mentionnée ci-dessus pour la détermination de la teneur globale en fer d'un lubrifiant circulant dans un moteur de navire.

L'invention sera mieux comprise et d'autres avantages de celle-ci apparaitront plus clairement à la lumière de la description qui va suivre de deux modes de réalisation d'une installation conforme à son principe, donnée uniquement à titre d'exemple et faite en référence aux dessins annexés dans lesquels :
- la figure 1 est une représentation schématique de principe d'une installation conforme à l'invention telle qu'embarquée sur un navire,
- la figure 2 est une vue à plus grande échelle du détail II à la figure 1, montrant un capteur de détermination de la teneur globale en un élément chimique prédéterminé, notamment en fer, utilisé dans l'installation de la figure 1.
- la figure 3 est une vue à plus grande échelle du détail III à la figure 2,
- la figure 4 est une vue en perspective du capteur représenté aux figures 2 et 3,
- la figure 5 est une vue en perspective, selon un autre angle, du capteur des figures 2 à 4,
- la figure 6 est une représentation schématique à plus petite échelle de la partie fluidique de l'installation de la figure 1 dans une première configuration d'utilisation,
- les figures 7 à 9 sont des vues analogues à la figure 6 lorsque l'installation est dans une deuxième, une troisième et une quatrième configuration d'utilisation,
- la figure 10 est une vue analogue à la figure 1 pour une installation conforme à un deuxième mode de réalisation de l'invention,
- les figures 11 à 21 sont des vues analogues à la figure 6 pour l'installation de la figure 10 dans différentes configurations d'utilisation, et
- la figure 22 est une vue analogue à la figure 1 pour une installation conforme à un troisième mode de réalisation de l'invention.

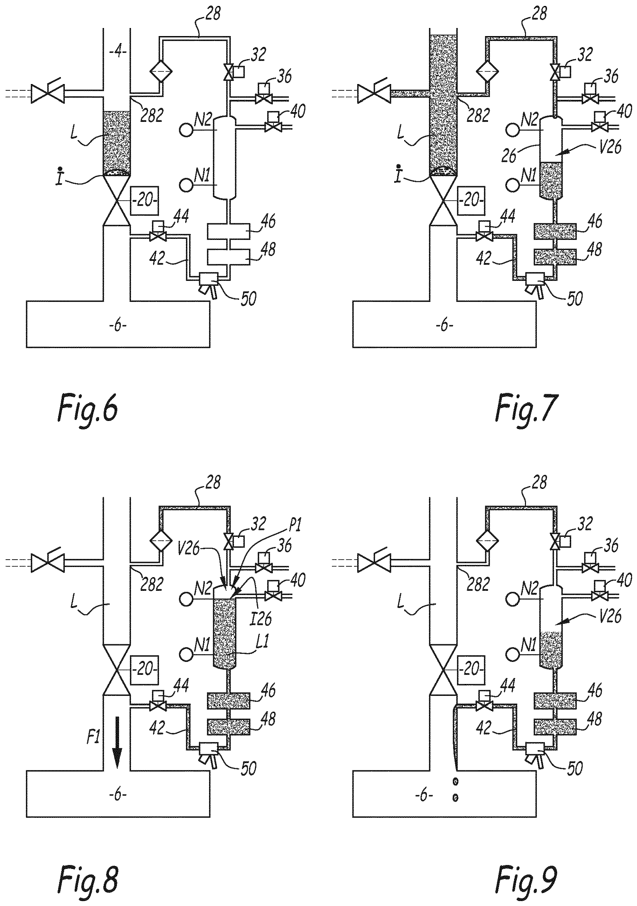

Sur les figures 6 à 9 et 11 à 21, le lubrifiant présent ou circulant dans une partie de l'installation est représenté en grisé.

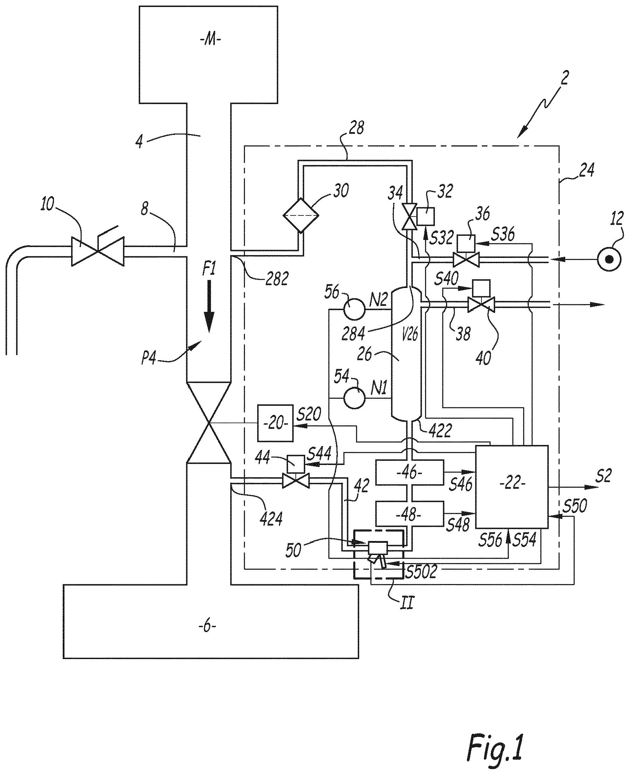

L'installation 2 représentée aux figures 1 à 9 est embarquée sur un navire représenté à la figure 1 par un équipement M. En particulier, l'équipement M est un moteur qui comporte plusieurs cylindres, par exemple de deux à quatorze cylindres, de préférence un moteur du navire à deux temps ou quatre temps. Une conduite 4 relie l'équipement M à un bac 6 de récupération de lubrifiant. En pratique, l'huile moteur s'écoule dans la conduite 4 avec une pression P4 comprise entre 1,1 et 6 bars absolus. Le débit d'huile dans la conduite 4 peut être faible, au point que l'huile ruisselle sur la paroi interne de cette conduite.

La conduite 4 s'étend verticalement, de haut en bas, de l'équipement M vers le bac 6. Dans ce mode de réalisation, l'huile s'écoulant dans la conduite 4 provient d'au moins un cylindre de l'équipement M.

Un piquage 8 est prévu sur la conduite 4 et équipé d'une vanne 10 commandée manuellement, ce qui permet de prélever une quantité d'huile sortant de l'équipement M afin de procéder à des analyses physico-chimiques, selon une approche connue en soi.

L'installation 2 comprend une vanne d'arrêt 20 montée sur la conduite 4 et qui permet d'interrompre, de façon sélective, l'écoulement d'huile dans la conduite 4, en direction du bac 6. La vanne d'arrêt 20 est commandée par une unité électronique 22 au moyen d'un signal électrique S20.

Comme visible uniquement à la figure 1, l'installation 2 comprend un coffret 24, représenté par sa trace en trait d'axe et à l'intérieur duquel sont disposés les éléments constitutifs de l'installation 2, à l'exception de la partie de la vanne d'arrêt 20 qui est intégrée à la conduite 4.

L'installation 2 comprend également un réservoir tampon 26 qui est disposé dans le coffret 24 et qui est relié à la conduite 4 au moyen d'une première ligne de dérivation 28.

On note 282 l'embouchure de la ligne 28. Cette embouchure est disposée en amont de la vanne 20 sur la conduite 4. La première ligne de dérivation 28 est équipée, en aval de son embouchure 282 vers son débouché 284 dans le réservoir tampon 26, d'un filtre 30, d'une vanne d'arrêt 32 et d'un piquage 34. Le filtre 30 sert à empêcher des impuretés de taille trop importante de s'écouler dans la première ligne de dérivation 28. La vanne d'arrêt 32 permet, au choix, de rendre passante ou de fermer la première ligne de dérivation 28. La vanne 32 est commandée par l'unité électronique 22, au moyen d'un signal électrique S32. Le piquage 34 est relié, à travers une vanne commandée 36, à une source 12 d'air sous pression qui ne fait pas partie de l'installation 2 mais appartient aux équipements standards d'un navire.

En pratique, la source 12 d'air sous pression peut être un compresseur embarqué sur le navire et qui alimente un réseau d'air comprimé qui sert également à d'autres équipements que l'installation 2. En variante, la source 12 peut être une pompe dédiée à l'installation 2.

L'installation 2 comprend également un piquage 38 raccordé au réservoir 26, sur lequel est montée une vanne d'arrêt 40 et qui permet de mettre en communication le volume intérieur V26 du réservoir 26 avec l'atmosphère ambiante.

Dans ce mode de réalisation, les piquages 34 et 38 sont indépendants. En variante, ils peuvent être remplacés par un unique piquage, raccordé à la première ligne 28 ou directement au réservoir 26, sur lequel les vannes 36 et 40 sont montées en parallèle, en étant respectivement reliées à la source 12 d'air sous pression et à l'atmosphère ambiante. Dans ce cas, il est possible de combiner les vannes 36 et 40 sous la forme d'une unique vanne trois voies.

Les vannes 36 et 40 sont commandées par l'unité électronique 22 au moyen de signaux électriques respectifs S36 et S40.

L'installation 2 comprend également une deuxième ligne 42 d'évacuation du lubrifiant, du volume intérieur V26 du réservoir 26 vers le bac de récupération 6. La deuxième ligne d'évacuation 42 est donc disposée en aval de la première ligne de dérivation 28 et du réservoir 26, sur le trajet d'écoulement du lubrifiant. Dans l'exemple, la deuxième ligne 42 s'étend du réservoir 26 vers la conduite 4. Son embouchure 422 est située en partie basse du réservoir 26, alors que son débouché 424 est disposé sur la conduite 4, en aval de la vanne d'arrêt 20, comme représenté sur les figures, ce qui permet de réduire le temps d'un cycle d'analyse car la vanne d'arrêt 20 peut être fermée pour créer une colonne d'huile dans la conduite 4, alors que des étapes de mesure ont lieu. En variante, le débouché 424 de la deuxième ligne 42 est disposé en amont de la vanne d'arrêt 20, ce qui permet d'exécuter simultanément des étapes de vidange et de décolmatage du filtre 30 et, éventuellement, de réduire le coût de l'installation 2.

La deuxième ligne 42 est équipée d'une vanne d'arrêt 44 qui est commandée par l'unité électronique 22 au moyen d'un signal électrique S44.

Trois capteurs 46, 48 et 50 sont disposés sur la ligne 42, en amont de la vanne 44.

Le capteur 46 permet de mesurer la densité D, la viscosité V, l'humidité H et la température T d'un lubrifiant, présent ou s'écoulant dans la deuxième ligne 42. Ce capteur peut être du type de celui commercialisé par la société AVENISENSE sous la dénomination Cactus. En variante, le capteur 46 peut être d'un autre type ou ne permettre de mesurer qu'un seul ou certains des paramètres mentionnés ci-dessus.

Le capteur 48 est un capteur d'indice de basicité ou BN, parfois dénommé indice d'alcalinité. Il peut s'agir d'un capteur fonctionnant avec la technologie infrarouge, dans le moyen infrarouge, ou de tout autre capteur adapté à la détermination du BN d'un lubrifiant.

Le capteur 50 est un capteur de détermination de la teneur globale en fer, c'est-à-dire la teneur en fer dissout et/ou particulaire, d'un échantillon de lubrifiant sortant du réservoir 26, au moyen de la technologie de fluorescence X.

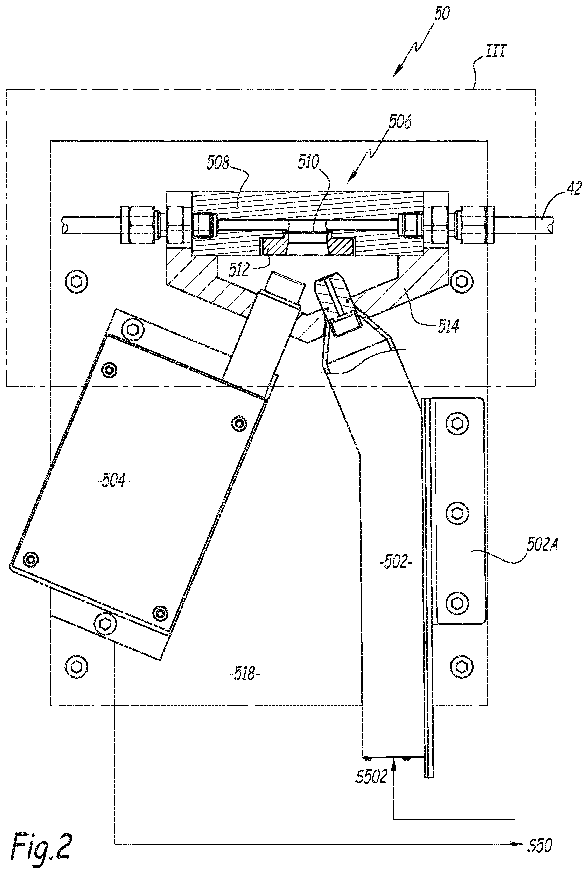

Comme cela ressort plus particulièrement des figures 2 à 5, le capteur 50 comprend une source 502 de rayon X, un détecteur 504 de rayon X et une cellule 506 qui est montée en série sur la deuxième ligne 42. Pour ce faire, la cellule 506 est pourvue d'un élément de raccord amont 506A qui coopère avec l'élément de raccord complémentaire 42A prévu sur la ligne 42, ainsi que d'un élément de raccord aval 506B qui coopère avec un élément de raccord complémentaire 42B prévu sur la ligne 42.

La source 502 comprend une cathode et une anode entre lesquelles circulent des électrons, sous l'effet d'une différence de potentiel de l'ordre de 50 kV, un courant de l'ordre de 500 mA circulant dans la cathode. L'anode est réalisée en métal, par exemple en or, en tungstène, en argent ou en rhodium. La puissance requise pour la source 502 est relativement modeste, notamment comprise entre 4 et 10 W. Un collimateur 502C est utilisé en sortie de la source 502 pour concentrer le faisceau d'électrons centré sur un axe de visée A502 de la source 502.

Les rayons émis par la source X sont dans la gamme des rayons X, avec une longueur d'onde comprise entre 0,01 et 10 nm, c'est-à-dire une fréquence comprise entre environ 3 x 10¹⁹ et 3 x 10¹⁶ Hz.

Le détecteur 504 est de type SDD (de l'Anglais Silicon Drift Detector) qui comprend une électrode unique en face avant, laquelle collecte les électrons générés par l'interaction des rayons X dans la jonction PN d'une photo diode. Ce type de détecteur présente l'avantage d'une faible capacitance due à la faible surface de son anode. Ce type de détecteur permet d'obtenir un taux de comptage élevé, une bonne résolution et un refroidissement efficace par effet Peltier. En variante, le détecteur 504 est de type SI-PIN, avec une photo diode en silicium, qui présente une zone intrinsèque intercalée entre ses deux zones respectivement dopées positivement et négativement.

Le détecteur 504 est capable de compter les « coups » d'émission à chaque niveau d'énergie sur une période donnée, ce qui permet d'établir un spectre de niveaux d'énergie. On note A504 l'axe de visée du détecteur 504, qui correspond à la direction principale des rayons X détectés par ce détecteur.

La cellule 506 comprend un corps 508 réalisé par usinage ou moulage d'un bloc de métal. Ce corps 508 est, de préférence, réalisé en aluminium ou en alliage à base d'aluminium, par exemple en Zicral (7075) qui est un alliage d'aluminium avec le zinc comme élément d'alliage principal. En variante, le boîtier 508 peut être réalisé dans un autre alliage à base d'aluminium. Au sens de la présente description, un alliage à base d'aluminium est un alliage qui comprend au moins 50% en poids d'aluminium. L'utilisation d'un alliage à base d'aluminium permet au boîtier 508 de résister à la température, à la pression et à la composition chimique du lubrifiant s'écoulant dans la ligne 42.L'absence ou la faible proportion de fer contenue dans cet alliage évite que la mesure par fluorescence X de la teneur globale en fer du lubrifiant soit perturbée par la présence de fer en quantité significative.

Toutefois, en variante, il est possible de prévoir que le boîtier 508 soit réalisé en acier inoxydable.

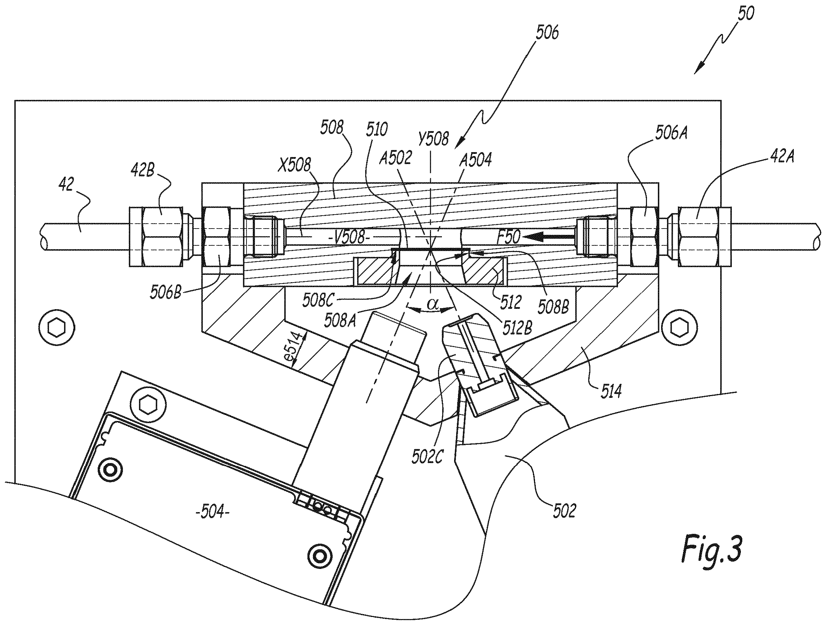

Le boîtier 508 définit un volume V508 de circulation du lubrifiant entre les éléments de raccord 506A et 506B, dans le sens de la flèche F50 à la figure 3. Ce volume V508 est de forme tubulaire à section circulaire ou rectangulaire, au choix du concepteur du boîtier 508. On note X508 un axe longitudinal du volume V508.

Le boîtier 508 est pourvu d'un perçage 508A, qui est centré sur un axe Y508 perpendiculaire à l'axe X508 et qui débouche dans le volume V508. Le perçage 508A est à section circulaire centrée sur l'axe Y508 et pourvu d'un taraudage 508B.

Le perçage 508A est obturé par une paroi 510 en forme de disque, que l'on peut également qualifier de « membrane » et qui est maintenue en appui contre le fond d'un lamage 508C du perçage 508A au moyen d'une bague 512 pourvue d'un filetage externe 512B complémentaire du taraudage 508B et qui coopère avec celui-ci. La paroi 510 est montée sur le boîtier 508 en la plaquant contre le lamage 508C, puis en vissant la bague 512 dans le perçage 508A.

La bague 512 est réalisée dans le même métal ou le même alliage que le corps 508.

La paroi 510 constitue une fenêtre de visée pour la source 502 et pour le détecteur 504 qui permet aux rayons X de transiter de la source 502 vers un échantillon de lubrifiant contenu dans le volume V508 et du volume 508 vers le détecteur 504.

La paroi 510 est réalisée en poly(téréphtalate d'éthylène) ou PET, ce qui lui confère des propriétés mécaniques satisfaisantes, alors qu'elle peut avoir une épaisseur faible, inférieure à 200 µm, au point qu'elle ne perturbe pas les rayons X qui proviennent de la source 502 ou qui partent vers le détecteur 504.

En pratique, l'épaisseur de la paroi 510, qui est mesurée perpendiculairement à l'axe Y508, peut être choisie inférieure à 150 µm, de préférence de l'ordre de 125 µm.

La source 502 et le détecteur 504 sont montés sur un capot 514 qui détermine leur position par rapport à la cellule 506, plus particulièrement par rapport au boîtier 508 et à la paroi 510. Ce capot 514 entoure le boîtier 508 du côté du perçage 508A, de sorte qu'il isole la fenêtre formée par la paroi 510 de l'extérieur du capteur 50. On note e514 l'épaisseur du capot 514. Le matériau de ce capot 514 et son épaisseur e514 sont choisis de telle sorte qu'ils constituent un blindage efficace contre les rayons X qui circulent entre la source 502, le détecteur 504 et la cellule 506. Cette cellule permet le libre passage du lubrifiant et permet d'effectuer des analyses de lubrifiant fluide statiques ou dynamiques. En pratique, le capot 514 peut être réalisé en inox, par exemple de type 316, et l'épaisseur e514 est choisie supérieure à 5 mm, de préférence supérieure à 8 mm, de préférence encore de l'ordre de 10 mm.

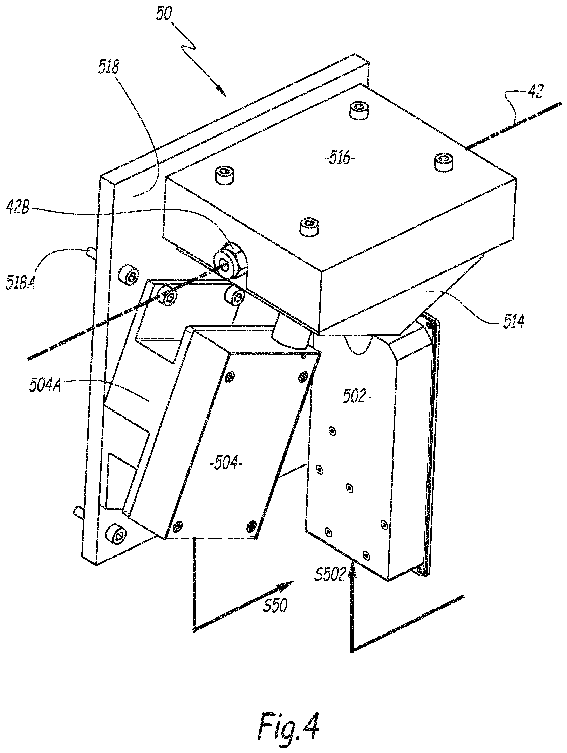

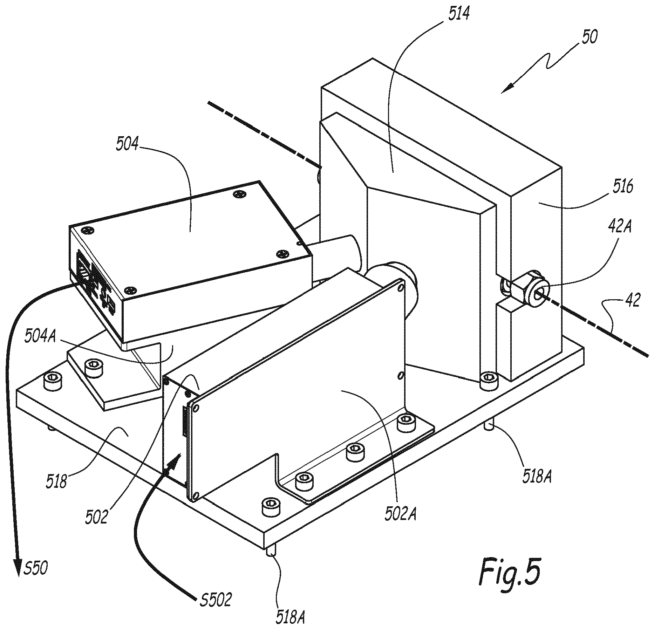

D'autre part, un blindage complémentaire 516 est monté autour du boîtier 508, du côté de ce boîtier opposé au capot 514. Pour la clarté du dessin, ce blindage 516 est représenté uniquement aux figures 4 et 5.

Les parties 502, 504, 506, 514 et 516 du capteur 50 sont fixées sur un support 518 constitué par une plaque qui peut être immobilisée dans le coffret 24 au moyen de vis 518A. Une équerre 502A et une entretoise 504A sont utilisées pour fixer respectivement la source 502 et le détecteur 504 sur le support 518.

Ainsi assemblé, le capteur 50 constitue un sous-ensemble facile à manipuler, aisément identifiable, et qui peut faire l'objet d'une opération d'échange standard, en dévissant les vis 518A et en séparant les éléments de raccord 506A et 42A, d'une part, 506B et 42B, d'autre part.

La source 502 est commandée par l'unité électronique 22 au moyen d'un signal S502 et le détecteur 504 délivre à l'unité électronique 22 un signal de sortie S50 du capteur 50.

En pratique, les axes X508, Y508, A502 et A504 sont coplanaires.

On note α un angle mesuré entre les axes A502 et A504 à l'extérieur du boîtier 508. Cet angle α a une valeur comprise entre 20 et 25°, de préférence de l'ordre de 22°.

En fonctionnement, la technologie de fluorescence X du capteur 50 est utilisée pour déterminer la teneur globale en fer, c'est-à-dire la teneur en fer dissout et/ou particulaire, d'une quantité d'huile transitant dans le volume V508, en sortie du réservoir tampon 26.

La mesure de la teneur en fer effectuée par le capteur 50 lorsque le lubrifiant s'écoule dans la deuxième ligne 42, c'est-à-dire lorsque la vanne 44 est ouverte. On parle alors de mesure dynamique.

En variante, cette mesure peut être effectuée lorsque le lubrifiant est statique dans le volume V508, c'est-à-dire lorsque la vanne 44 est fermée. On parle alors de mesure statique.

L'installation 2 comprend également un premier capteur de niveau 54 et un deuxième capteur de niveau 56 qui permettent respectivement de détecter lorsque la quantité de l'huile dans le réservoir 26 atteint un premier niveau N1 ou un deuxième niveau N2. Les signaux électriques de sortie S54 et S56 des capteurs 54 et 56 sont délivrés à l'unité 22.

En variante, les capteurs 54 et 56 peuvent être remplacés par un unique capteur, tel qu'un capteur de pression, qui permet de détecter lorsque l'huile atteint chacun des deux niveaux N1 et N2 dans le réservoir 26.

Les figures 6 à 9 illustrent schématiquement les étapes successives d'un procédé automatisé mis en oeuvre grâce à l'installation 2 de la figure 1. Ce procédé est automatisé en ce sens qu'il est peut être implémenté, partiellement ou de préférence totalement, sans intervention humaine, sous le contrôle de l'unité 22. Il en va de même pour le procédé explicité ci-après au sujet du deuxième mode de réalisation de l'invention.

Par défaut, et en dehors des phases de prélèvement, l'huile sortant de l'équipement M s'écoule dans la conduite 4, dans le sens de la flèche F1 à la figure 1, de l'équipement M vers le bac de récupération 6, sans être retenue par la vanne 20 qui est en configuration ouverte ou passante, alors que les autres vannes sont fermées.

Lorsqu'il convient de déterminer la teneur en fer de l'huile sortant du moteur M, l'unité 22 pilote la vanne 20 à la fermeture, de sorte qu'il est créé dans la conduite 4 une retenue où s'accumule une quantité d'huile, c'est-à-dire de lubrifiant, comme représenté par la partie grisée L à la figure 2.

Dans la configuration de la figure 6, la conduite 4 sert de colonne de décantation et des impuretés 1 s'accumulent au voisinage de la vanne 20, à l'intérieur de la conduite 4 et en partie basse de la quantité de lubrifiant L.

Dans cette première étape représentée par la configuration de la figure 6, les vannes 32 et 40 sont ouvertes, alors que les vannes 36 et 44 sont fermées.

Lorsque le niveau de lubrifiant L dans la conduite ou colonne 4 atteint l'embouchure 282, de l'huile commence à s'écouler à travers la première ligne de dérivation 28, plus particulièrement à travers le filtre 30 et la vanne 32, jusque dans le volume intérieur V26 du réservoir 26 dans lequel l'huile s'écoule. En effet, le débouché 284 de la première ligne 28 est situé en partie haute du réservoir 26 et l'huile peut s'écouler le long de la paroi du réservoir 26. Comme la vanne 44 est fermée, l'huile remplit progressivement la partie de la deuxième ligne d'évacuation 42 située en amont de la vanne 44, y compris les volumes internes des capteurs 46 et 48, puis le volume intérieur V26, en chassant l'air vers l'atmosphère, à travers la vanne 40. Cette étape correspond à la configuration représentée à la figure 7.

Lorsque le capteur 56 détecte que le niveau N2 d'huile à l'intérieur du réservoir 26 est atteint, l'unité 22 bascule l'installation 2 vers une nouvelle configuration représentée à la figure 8, dans laquelle la vanne 20 passe en configuration ouverte, ce qui permet de vidanger la colonne de décantation en dirigeant le reliquat de la quantité L de lubrifiant présente en amont de la vanne 20 ainsi que les impuretés 1 vers le bac de récupération 6. L'écoulement dans le sens de la flèche F1 se poursuit donc jusque dans le bac 6. Par ailleurs, les vannes 32 et 40 sont fermées et la vanne 36 est ouverte, ce qui permet de mettre la partie du volume V26 qui n'est pas occupée par le lubrifiant, c'est-à-dire la partie de ce volume V26 située au-dessus du niveau N2, sous une pression d'air P1 égale à celle de la source d'air 12, qui, dans l'exemple, vaut 7 bars absolus.

Ceci étant fait, l'unité 22 fait passer l'installation 2 à une étape suivante, représentée par la configuration de la figure 9, où la vanne 44 est ouverte, les autres vannes conservant leur état de la configuration de la figure 4. Dans ce cas, la pression P1 de l'air dans la partie supérieure du volume V26 a pour effet de pousser l'huile dans la deuxième ligne d'évacuation 42, à travers les capteurs 46, 48 et 50, ce qui permet à ces capteurs de fournir à l'unité 22 des signaux S46, respectivement S48 et S50, représentatifs des paramètres qu'ils ont détectés.

Le cas échéant, les signaux S46, S48 et S50 peuvent être traités dans l'unité 22 pour déterminer les valeurs des paramètres contrôlés, notamment par comparaison avec des valeurs connues pour des lubrifiants de référence.

Les signaux S46, S48 et S50, ou des signaux extrapolés à partir de ces signaux, peuvent être fournis à l'extérieur de l'installation 2 sous la forme d'un signal conjugué S2, exploitable par une unité centrale de contrôle de l'équipement M.

En pratique, la section de passage du capteur 50 de teneur en fer est d'environ 70 mm². Elle peut aller jusqu'à 200 mm². Dans tous les cas, il convient de pouvoir alimenter cette section de passage avec un débit suffisant, pendant une durée suffisante à la réalisation de la mesure la teneur globale en fer. En variante, il en va de même pour le capteur 46 et le capteur 48 d'indice de basicité. La construction de l'installation avec le réservoir 26 permet de créer une réserve formant un « tampon » d'huile, sous la forme de la quantité d'huile L1 contenue dans le réservoir 26 dans la configuration de la figure 4. Une partie de cette réserve d'huile L1 peut être déversée, de façon continue ou séquentielle, dans la deuxième ligne d'évacuation 42 pour que les capteurs 48 et 50 disposent d'une quantité suffisante d'huile à analyser.

A partir de la configuration de la figure 9, il est possible, dans une étape subséquente, de poursuivre la vidange du réservoir 26 et de l'intégralité de la deuxième ligne d'évacuation 42 en maintenant la vanne 44 ouverte et en poursuivant l'injection d'air comprimé à travers la vanne 36.

En variante, il est possible d'arrêter la vidange du réservoir 26 lorsque le niveau d'huile atteint le niveau N1, de façon à conserver en permanence une quantité L2 d'huile dans la deuxième ligne d'évacuation 42, en particulier dans les capteurs 46, 48 et 50 dont les parties actives au contact de l'huile ne risquent pas de sécher. Ceci évite notamment le dépôt de traces d'huile sur la paroi 510 du capteur 50. Si cette deuxième approche est sélectionnée, une certaine quantité d'huile doit être utilisée lors d'une prochaine mesure, pour nettoyer préalablement la deuxième ligne d'évacuation 42 et ne pas perturber la prochaine mesure.

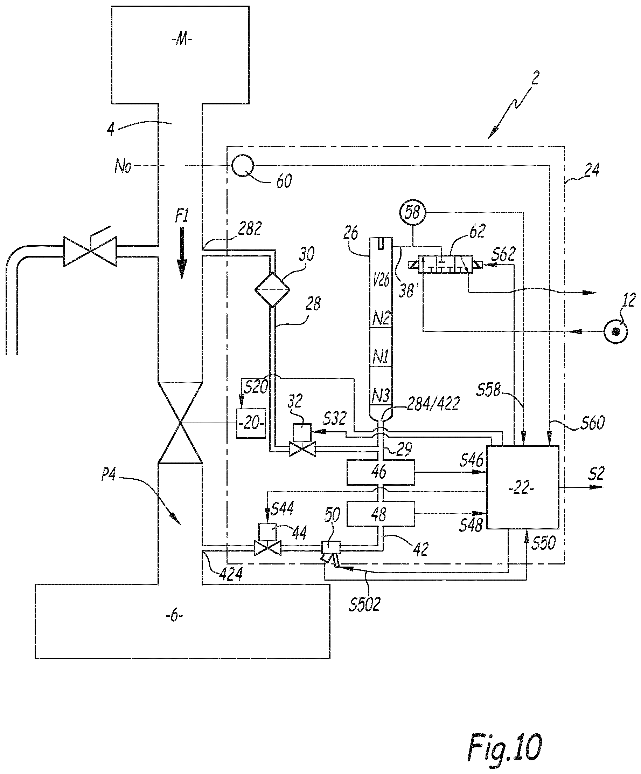

Dans les deuxième et troisième modes de réalisation de l'invention représentés aux figures 10 et suivantes, les éléments analogues à ceux du premier mode de réalisation portent les mêmes références. Dans ce qui suit, on décrit principalement ce qui distingue ces modes de réalisation du précédent.

Dans le mode de réalisation des figures 10 à 21, les première et deuxième lignes 28 et 42 se rejoignent au niveau d'un embranchement 29 en T. Ainsi, le débouché 284 de la première ligne de dérivation 28 est confondu avec l'embouchure 422 de la deuxième ligne d'évacuation 42. Le tronçon de ligne situé entre le réservoir 26 et l'embranchement 29 est commun aux première et deuxième lignes 28 et 42. Ce tronçon de ligne débouche en partie basse du réservoir 26, de sorte que l'huile qui s'écoule de la conduite 4 vers le réservoir 26 parvient directement en partie basse de ce réservoir.

On définit trois niveaux N1, N2 et N3 dans le réservoir 26, les niveaux N1 et N2 étant comparables à ceux du premier mode de réalisation.

Dans ce deuxième mode de réalisation, il n'est pas utilisé de capteurs de niveau identiques aux capteurs de niveaux 54 et 56, mais un capteur de pression 58 dont le signal de sortie S58 est fourni à l'unité de contrôle électronique 22. Par ailleurs, un capteur de niveau 60 est monté dans la conduite 4, en amont de la vanne 20, c'est-à-dire au-dessus de celle-ci. Il fournit à l'unité 22 un signal S60.

En outre, les piquages 34 et 38 et les vannes 36 et 40 du premier mode de réalisation sont remplacés par un unique piquage 38' sur lequel est branché le capteur de pression 58, ainsi qu'un distributeur 62 à trois voies et trois orifices, qui est raccordé d'une part à la source d'air sous pression 12 et d'autre part à l'atmosphère ambiante. Le distributeur 62 est commandé par l'unité 22 au moyen d'un signal électrique dédié S62.

L'installation 2 comprend, comme dans le premier mode de réalisation, un capteur 50 de détermination de la teneur globale en fer, c'est-à-dire la teneur en fer dissout et/ou particulaire, identique à celui du premier mode de réalisation et monté sur la conduite 42.

Le fonctionnement de l'installation 2 est le suivant :
Par défaut, la vanne 20 est ouverte et les vannes 32 et 44 sont fermées, alors que le distributeur 62 est dans la configuration représentée à la figure 10 où il isole le volume intérieur V26 du réservoir 26 de la source 12 d'air comprimée et de l'atmosphère ambiante.

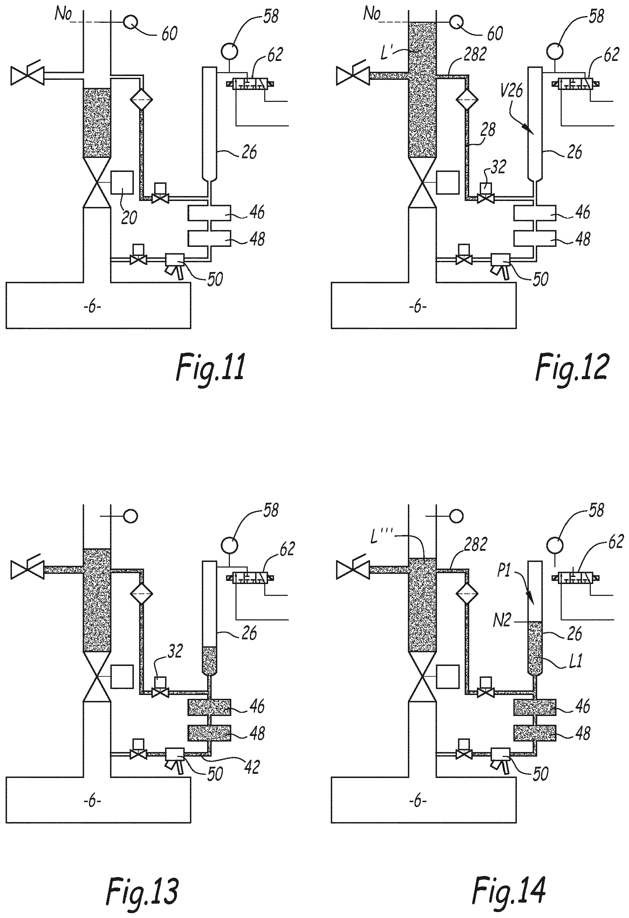

Lorsqu'il convient de procéder à la détermination de la teneur en fer et optionnellement de l'indice de basicité de l'huile sortant de l'équipement M, l'unité 22 active la vanne 20 au moyen du signal S20 dans une première étape, pour l'amener en configuration fermée représentée à la figure 11. Dans cette configuration, de l'huile est présente dans la première ligne de dérivation 28, entre le filtre 30 et la vanne 32, du fait d'une opération de décolmatage du filtre 30, effectuée précédemment et qui est explicité ci-après.

Dans cette configuration, les vannes 32 et 44 et le distributeur 62 sont fermés.

Le capteur de niveau 60 est positionné pour que, lorsque la colonne d'huile retenue dans la conduite 4 en amont de la vanne 20 atteint le niveau N0 détecté par ce capteur, comme représenté à la figure 12, une quantité prédéterminée de lubrifiant est présente au-dessus de l'embouchure 282. Par exemple, la quantité prédéterminée peut être égale à 100 ml. Lorsque le capteur de niveau 60 détecte que ce niveau N0 est atteint dans la conduite 4, le volume intérieur V26 du réservoir 26 est mis à la pression atmosphérique en actionnant le distributeur 62 pour l'amener dans la configuration de la figure 12.

A partir de cette configuration, l'unité 22 commande la vanne 32 et le distributeur 62 dans une étape suivante pour réaliser le transfert de la quantité d'huile de la conduite 4 vers le réservoir 26, comme représenté par la configuration de la figure 13. Dans cette configuration, la vanne 32 est ouverte, alors que le distributeur 62 est fermé. Le transfert d'huile de la conduite 4 vers le réservoir tampon 26 s'accompagne donc d'une augmentation de la pression d'air à l'intérieur du réservoir 26. Le taux de compression de l'air piégé dans le réservoir peut être relié, après étalonnage, au volume initial d'air dans le réservoir 26 et au volume d'huile transvasé.

Par exemple, pour une compression adiabatique et un volume d'air initial dans le réservoir 26 égal à 160 ml, la pression dans le réservoir 26 atteint 1,7 bar absolu pour 50 ml d'huile transférés.

De même, en considérant un réservoir 26 contenant initialement 250 ml d'air, il est possible de transférer 80 ml, soit la quantité L1 représentée à la figure 10, dans le réservoir 26 avant d'atteindre en partie supérieure de celui-ci une pression d'air P1 égale à 1,7 bar absolu. C'est l'exemple considéré dans ce qui suit.

Dans ce cas, le niveau d'huile N2 est atteint dans le réservoir 26 au niveau de l'étape représentée par l'installation 2 dans la configuration de la figure 14.

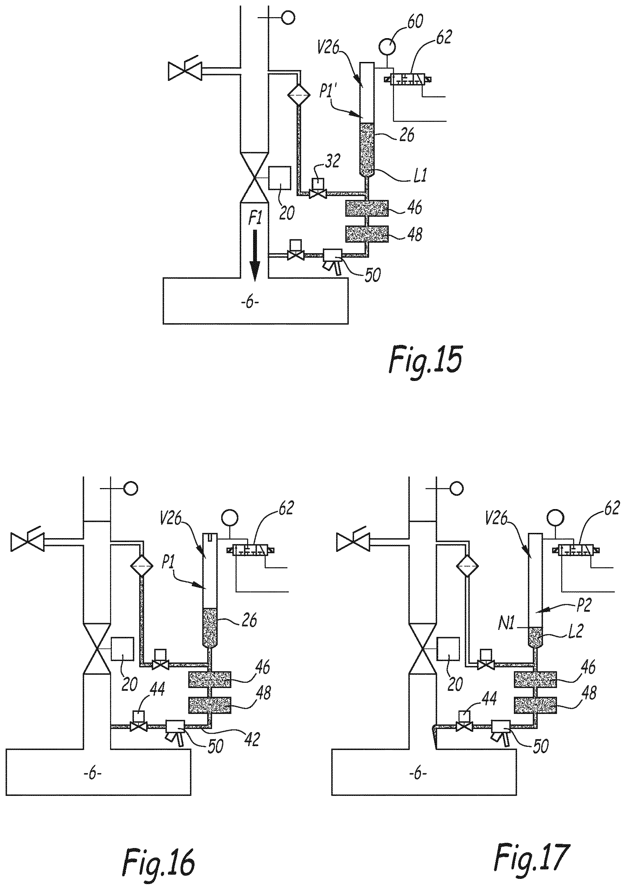

L'unité 22 commande alors automatiquement les vannes et le distributeur pour atteindre la configuration de la figure 15 où le réservoir 26 est mis sous pression à travers le distributeur 62 qui raccorde le volume V26 à la source d'air comprimé 12, de sorte que la pression P1' d'air à l'intérieur du réservoir 26 devient égale à 7 bars. Pour que ceci puisse avoir lieu, la vanne 32 a préalablement été basculée par l'unité 22 en configuration fermée, afin d'éviter un retour d'huile du réservoir 26 vers la canalisation 4. Par ailleurs, dans cette étape, la vanne 20 est basculée par l'unité 22 en configuration ouverte, de sorte que l'écoulement d'huile du moteur circulant dans l'équipement M vers le bac de récupération 6 peut à nouveau avoir lieu dans le sens de la flèche F1. A partir de cette configuration, l'unité 22 contrôle le distributeur 62 et la vanne 44 au moyen des signaux S62 et S44, respectivement pour fermer le distributeur 62 et ouvrir la vanne 44 et atteindre ainsi la configuration de la figure 16 où l'huile contenue dans le réservoir 26 est progressivement chassée de celui-ci du fait de la pression P1 régnant en partie supérieure du volume intérieur V26.

L'huile s'écoule donc à travers les capteurs 46, 48 et 50 qui sont capables de détecter les paramètres pour lesquels ils sont prévus et de fournir des signaux correspondants, S46, S48 et S50, à l'unité 22, comme dans le premier mode de réalisation.

La décharge de l'huile contenue dans le réservoir 26 à travers la deuxième ligne d'évacuation 42 peut avoir lieu en plusieurs cycles, par détentes successives du volume d'air piégé dans le réservoir et connexions successives à la source d'air 12. Pour un réservoir de 250 ml contenant initialement 80ml d'huile, on peut par exemple effectuer trois détentes successives, entre 7 bars et 6,2 bars, précédées de trois connexions à la source d'air 12. Ceci permet de délivrer un volume total de 50 ml dans la deuxième ligne d'évacuation 42 et d'atteindre la configuration de la figure 17 où une quantité résiduelle L2, de 30 ml de lubrifiant, demeure dans le réservoir 26 en étant soumise à une pression P2 égale à 6,2 bars.

Les trois détentes successives ont lieu en remplissant préalablement et successivement le réservoir 26 avec de l'air à 7 bars, au moyen d'une commande appropriée du distributeur 62.

Ces trois détentes permettent de faire circuler 50 ml de lubrifiant dans les capteurs 46, 48 et 50 en trois étapes successives, ce qui leur permet de générer trois jeux de signaux S46, S48 et S50 ou un jeu de signaux combinés, destiné(s) à et fourni(s) à l'unité 22, puis transmis et/ou traité(s) comme dans le premier mode de réalisation.

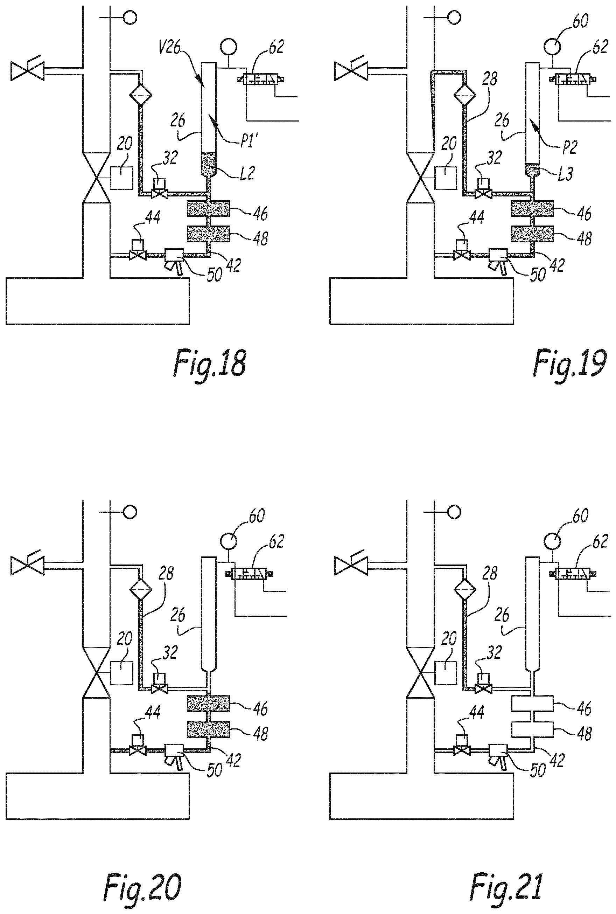

A partir de la configuration de la figure 17, l'unité 22 fait passer l'installation 2 dans la configuration de la figure 18 où le volume intérieur V26 du réservoir 26 est à nouveau pressurisé à la pression P1' de 7 bars, moyennant une commande appropriée du distributeur 62, alors que la vanne 44 est fermée.

Une fois cette opération réalisée, l'unité 22 commande la vanne 32 à l'ouverture et le distributeur 62 à la fermeture, ce qui a pour effet de chasser l'huile présente en partie basse du réservoir 26 à travers la première ligne de dérivation 28, jusque dans la conduite 4, dans un sens de décolmatage du filtre 30. Cette étape est représentée par la configuration de la figure 19. Le fait de faire baisser la pression dans le réservoir 26 de 7 à 6,2 bars permet de faire circuler une quantité d'environ 20 ml du réservoir 26 vers la conduite 4. Au terme de cette étape, il reste une quantité L3 égale à 10 ml de lubrifiant dans le réservoir 26, sous une pression P2 de 6,2 bars.

Une fois cette opération de décolmatage du filtre réalisé, l'unité 22 fait passer l'installation 2 dans la configuration de la figure 20 où la vanne 32 est à nouveau fermée, alors que la vanne 44 est ouverte et que le distributeur 62 est placé dans une configuration d'alimentation du volume V26 en air sous pression. Ceci a pour effet d'évacuer la quantité résiduelle d'huile présente dans la deuxième ligne d'évacuation 42 et dans les capteurs 46 et 48 jusqu'à obtenir la configuration de la figure 21 où la deuxième ligne d'évacuation 42 et les capteurs 46 et 48 sont vides d'huile et remplis d'air. Ceci correspond à la configuration de la figure 11 mentionnée ci-dessus.

On remarque sur les figures 20 et 21 que la partie de la première ligne de dérivation 28 située entre la vanne 32 et le débouché 284 est vidée par l'air provenant du réservoir 26. Ceci est à rapprocher du fait que, en pratique, la vanne 32 est disposée immédiatement en amont de l'embranchement 29.

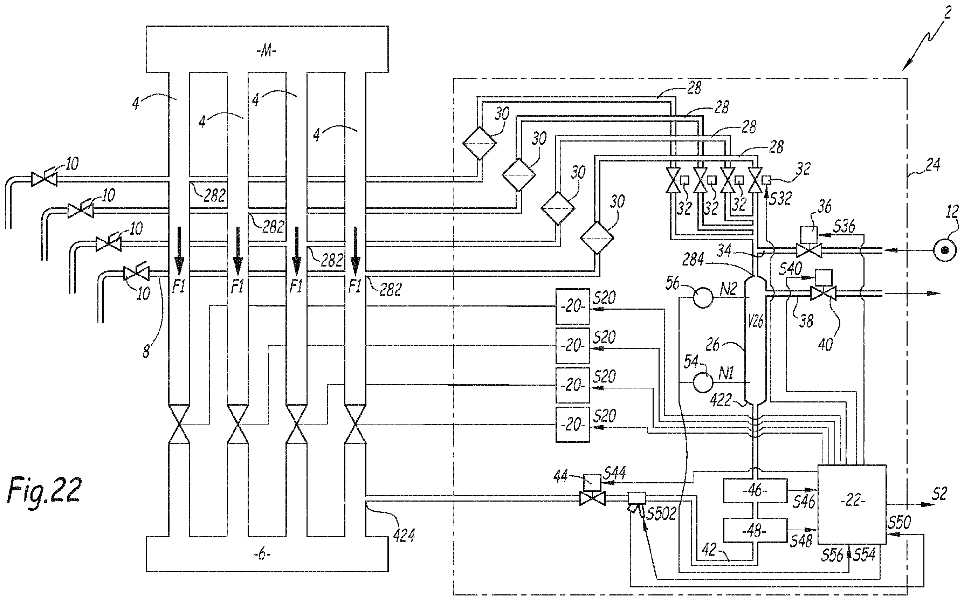

Dans le troisième mode de réalisation de l'invention représenté à la figure 22, plusieurs conduites 4 sont utilisées, chacune d'entre elle étant prévue pour collecter de l'huile à partir d'un unique cylindre de l'équipement M.

Chaque conduite 4 est équipée d'une vanne 20 commandée par l'unité électronique 22 et qui permet d'interrompre l'écoulement d'un flux F1 de lubrifiant dans la conduite 4 considérée. Une première ligne de dérivation 28 est raccordée, d'une part, à chaque conduite 4, en amont de sa vanne 20 et, d'autre part, à l'entrée du réservoir tampon 26 qui est le même que dans le premier mode de réalisation. L'installation 2 comprend donc autant de premières lignes de dérivation 28 que de conduites 4. En partant de son embouchure 282, chaque première ligne de dérivation 28 est équipée d'un filtre 30 et d'une vanne d'arrêt 32. Les quatre premières lignes 28 se rejoignent en aval de leurs vannes d'arrêt 32 respectives et le piquage 34 est commun aux quatre premières lignes de dérivation 28, de même que leur débouché 284 dans le réservoir tampon 26.

Un piquage 8 est prévu sur chaque conduite 4 et équipé d'une vanne 10 commandée manuellement, selon une approche parallèle à celle mentionnée ci-dessus au sujet du premier mode de réalisation. En variante, seule une ou certaines conduites 4 est ou sont équipées d'un tel piquage 8.

La deuxième ligne d'évacuation 42 est commune pour tous les cylindres de l'équipement, en particulier du moteur, et reçoit, en aval du réservoir 26, l'huile provenant de toutes les premières lignes de dérivation 28. Le débouché 424 de la deuxième ligne d'évacuation est disposé sur l'une des conduites 4, en aval de sa vanne d'arrêt 20.

Ce troisième mode de réalisation permet d'optimiser l'encombrement des conduites 4 et leur cheminement au sein du compartiment moteur d'un navire. Elle permet un gain de place par rapport au premier mode de réalisation.

En mettant en oeuvre successivement la méthode explicitée ci-dessus au sujet du premier mode de réalisation, pour chacune des conduites 4, l'installation de ce troisième mode de réalisation permet de connaître, grâce au capteur 50, identique à celui du premier mode de réalisation, la teneur globale en fer du lubrifiant en sortie de chacun des cylindres de l'équipement M sur lequel est raccordée une conduite 4.

Dans l'exemple de la figure 19, quatre conduites 4 sont prévues, chacune dédiée à un cylindre de l'équipement M. En variante, le nombre de conduites 4 est différent, tout en restant supérieur ou égal à 2, afin d'adapter l'installation 2 en fonction de la configuration de l'équipement M et de la place disponible pour loger les conduites 4.

En variante, l'invention peut être représentée avec les capteurs 46, 48 et 50 disposés dans cet ordre le long de la ligne d'évacuation 42. Ceci n'est pas obligatoire et un autre ordre est envisageable. Par exemple, le capteur 50 peut être disposé en amont des capteurs 46 et 48, ou entre ceux-ci, le long de la ligne 42.

Quel que soit le mode de réalisation, les capteurs 46 et 48 sont optionnels, en ce sens qu'ils ne sont pas utiles pour déterminer la teneur globale en fer de l'huile. Ils sont toutefois très avantageux car ils permettent à l'installation 2 de réaliser automatiquement plusieurs mesures de plusieurs paramètres représentatifs de la qualité de l'huile, en plus de la seule teneur en fer dissout et/ou particulaire. Ainsi, l'installation 2 permet une mesure efficace de la teneur globale en fer, de l'indice de basicité ou BN et/ou d'autres paramètres d'une huile sortant de l'équipement M grâce à un procédé qui peut être automatisé et qui ne nécessite pas de connaissance particulière de la part d'un utilisateur ou personnel naviguant, puisque le signal S2 peut être directement lisible, soit par l'homme soit par une machine.

En pratique, la pression maximum P1' régnant dans le volume intérieur V26 du réservoir 26, qui dépend de la pression de la source 12, n'est pas limitée à 7 bars. Elle est comprise entre 6 et 12 bars, de préférence entre 7 et 10 bars en fonction de la pression du réseau d'air comprimé disponible sur le navire. La valeur de 7 bars est préférée car elle donne de bons résultats expérimentaux et correspond à un niveau de pression couramment disponible. Il importe que cette pression P1 soit supérieure à la pression P4 de l'huile dans la conduite 4, qui est comprise entre 1,1 et 6 bars comme mentionné ci-dessus. En effet, c'est la différence entre les pressions P1 et P4 qui assure l'écoulement de l'huile à travers la deuxième conduite d'évacuation 42.

Quel que soit le mode de réalisation, l'installation 2, qui est pour l'essentiel comprise dans le coffret 24, est facile à installer à bord d'un navire et ne nécessite pas la mise en place de la vanne 20 dans la conduite 4, le branchement des lignes 28 et 42 sur cette conduite et son alimentation en courant et en air sous pression. L'installation 2 peut donc être aisément implantée sur un navire neuf ou utilisée pour rétrofiter un navire en service.

L'invention est décrite ci-dessus dans le cas où le capteur 50 sert à déterminer la teneur globale en fer du lubrifiant. Elle est toutefois applicable pour la détermination de la teneur globale d'un lubrifiant en un autre élément chimique prédéterminé, par exemple en calcium, vanadium, chrome, molybdène, soufre, plomb, cuivre, argent étain, aluminium, nickel, zinc ou en phosphore. Dans ce cas, le matériau constitutif du boîtier est adapté à l'élément chimique en question, pour ne pas perturber la mesure par fluorescence X.

L'invention est décrite ci-dessus dans le cas de son utilisation pour un moteur de propulsion de navire. Elle est toutefois applicable à d'autres équipements de navire, par exemple un moteur auxiliaire ou d'accessoire de navire.

Dans ce qui précède, les mots et expressions « huile », « huile moteur » et « lubrifiant » sont utilisés indistinctement car, au sens de l'invention, une huile ou une huile moteur est un lubrifiant.

Dans ce qui précède, les expressions « teneur en fer », « teneur globale en fer » et « teneur en fer dissout et/ou particulaire » sont utilisées indistinctement, de même que les expressions « teneur en élément chimique prédéterminé » et « teneur globale en élément chimique prédéterminé ».

Les caractéristiques des modes de réalisation et variantes envisagées ci-dessus peuvent être combinées pour générer de nouveaux modes de réalisation de l'invention.

## Revendications

1. Installation (2) de suivi de l'évolution de la qualité d'un lubrifiant circulant dans un équipement (M), cette installation comprenant :
- au moins une conduite (4) de circulation (F1) du lubrifiant, cette conduite étant raccordée, en amont, à l'équipement et, en aval, à un bac de récupération (6),
- un capteur (50) de détermination, par la technologie de fluorescence X, de la teneur globale en un élément chimique prédéterminé, d'un échantillon de lubrifiant, ce capteur comprenant une source de rayons X (502), un détecteur de rayons X (504) et une cellule (506) destinée à contenir un échantillon de lubrifiant à analyser et comprenant un boîtier (508),
- un réservoir (26) d'accumulation du lubrifiant;
- ledit capteur (50) étant configuré à déterminer la teneur globale en un élément chimique prédéterminé, d'un échantillon de lubrifiant en sortie du réservoir;
- une première vanne (20) d'interruption commandée de la circulation (F1) du lubrifiant dans la conduite (4);
- une première ligne (28) de dérivation raccordée, d'une part, à la conduite et, d'autre part au réservoir;
- une deuxième vanne (32) d'interruption commandée de la circulation du lubrifiant dans la première ligne de dérivation;
- une deuxième ligne (42) d'évacuation du lubrifiant, du réservoir vers le bac de récupération; et
- une paroi (510), qui forme une fenêtre de passage des rayons X issus de la source (502) ou allant vers le détecteur (504), réalisée en polymère;
**caractérisée**
• **en ce que** le réservoir est un réservoir tampon (26) d'accumulation du lubrifiant,
• **en ce que** l'installation comprend :
- la première ligne (28) de dérivation raccordée à la conduite en amont de la première vanne,
- une troisième vanne (44) d'interruption commandée de la circulation du lubrifiant dans la deuxième ligne d'évacuation,
• et
• **en ce que** le boîtier (508) est en métal
• **en ce que** paroi (510) est rapportée sur le boîtier (508) de la cellule (506) et
• **en ce que** la paroi (510) est réalisée en poly(téréphtalate d'éthylène).

2. Installation selon la revendication 1, **caractérisée en ce que** la cellule (506) comprend un logement creux (508C) de réception de la paroi (510) et une rondelle filetée (512) d'immobilisation de la paroi dans le logement creux.

3. Installation selon l'une des revendications 1 et 2, **caractérisée en ce que** le capteur (50) est un capteur de détermination de la teneur en fer de l'échantillon du lubrifiant.

4. Installation selon l'une des revendications 1 à 3, **caractérisée en ce que** le boîtier (508) est réalisé dans un métal ou un alliage non ferreux, notamment dans un alliage à base d'aluminium.

5. Installation selon l'une des revendications 1 à 4, **caractérisée en ce que** la source de rayons X (502) et le détecteur de rayons X (504) sont montés sur un capot (514) qui les positionne par rapport au boîtier (508) et à la paroi (510) et **en ce que** ce capot forme un blindage contre la propagation des rayons X.

6. Installation selon l'une des revendications précédentes, **caractérisée en ce qu'**un axe de visée (A502) de la source de rayons X (502) et un axe de visée (A504) du détecteur de rayons X (504) forment entre eux un angle (α) compris entre 20 et 25°, de préférence de l'ordre de 22°.

7. Installation selon l'une des revendications précédentes, **caractérisée en ce que** la paroi (510) a une épaisseur inférieure ou égale à 200 µm, de préférence inférieure ou égale à 150 µm, de préférence encore de l'ordre de 125 µm.

8. Installation selon l'une des revendications précédentes, **caractérisée en ce que** le capteur (50) de détermination de la teneur en un élément chimique prédéterminé est disposé sur la deuxième ligne d'évacuation (42).

9. Installation selon l'une des revendications précédentes, **caractérisée en ce qu'**elle comprend, en outre, un capteur (48) de détermination de l'indice de basicité (BN) du lubrifiant, disposé sur la deuxième ligne d'évacuation (42) et qui permet de déterminer l'indice de basicité du lubrifiant en sortie du réservoir tampon (26).

10. Procédé automatisé de suivi de l'évolution de la teneur en un élément chimique prédéterminé d'un lubrifiant circulant dans un équipement (M), au moyen d'une installation (2) selon la revendication 8, **caractérisé en ce qu'**il comprend au moins des étapes consistant à :
a) fermer la première vanne (20),
b) ouvrir la deuxième vanne (32) et fermer la troisième vanne (44) pour alimenter le réservoir tampon à partir d'une quantité (L ; L') de lubrifiant accumulée dans la conduite (4), en amont de la première vanne,
c) ouvrir la troisième vanne (44) pour faire circuler le lubrifiant présent dans le réservoir tampon (26) à travers la deuxième ligne d'évacuation (42) jusque dans une cellule du capteur (50).
d) utiliser le capteur (50) pour déterminer la teneur globale en un élément chimique prédéterminé du lubrifiant en sortie du réservoir tampon (26).

11. Méthode de suivi du fonctionnement d'un équipement (M) embarqué sur un navire, **caractérisée en ce qu'**elle comprend la détermination, à bord du navire, de la teneur globale en un élément chimique prédéterminé, notamment en fer, d'un lubrifiant circulant dans l'équipement par la mise en oeuvre d'un procédé selon la revendication 10.

12. Méthode selon la revendication 11 pour la détermination de la teneur globale en fer d'un lubrifiant circulant dans un moteur (M) de navire.

## Patentansprüche

1. Anlage (2) zur Überwachung der Änderung der Qualität eines Schmiermittels, das in einer Ausrüstung (M) zirkuliert, wobei diese Anlage umfasst:
- mindestens eine Leitung (4) für die Zirkulation (F1) des Schmiermittels, wobei diese Leitung stromaufwärts an die Ausrüstung und stromabwärts an ein Auffangbecken (6) angeschlossen ist,
- einen Sensor (50) zur Bestimmung des Gesamtgehalts eines vorbestimmten chemischen Elements in einer Probe mittels Röntgenfluoreszenztechnologie, wobei dieser Sensor eine Röntgenstrahlenquelle (502), einen Röntgenstrahlendetektor (504) und eine Zelle (506) zur Aufnahme einer zu analysierenden Schmiermittelprobe umfasst und ein Gehäuse (508) umfasst,
- einen Tank (26) zur Speicherung des Schmiermittels,
- wobei der Sensor (50) derart ausgelegt ist, dass er den Gesamtgehalt eines vorbestimmten chemischen Elements in einer Schmiermittelprobe am Auslass des Tanks bestimmt;
- ein erstes Ventil (20) zur gesteuerten Unterbrechung der Zirkulation (F1) des Schmiermittels in der Leitung (4);
- eine erste Bypassleitung (28), die einerseits an die Leitung und andererseits an den Tank angeschlossen ist;
- ein zweites Ventil (32) zur gesteuerten Unterbrechung der Zirkulation des Schmiermittels in der ersten Bypassleitung;
- eine zweite Leitung (42) zum Auslassen des Schmiermittels aus dem Tank in das Auffangbecken; und
- eine Wand (510), die ein Fenster für den Durchgang der Röntgenstrahlen bildet, die von der Quelle (502) kommen oder zum Detektor (504) gehen, die aus Polymer hergestellt ist;
**dadurch gekennzeichnet,**
• **dass** der Tank ein Puffertank (26) zur Speicherung des Schmiermittels ist,
• **dass** die Anlage umfasst:
- die erste Bypassleitung (28), die an die Leitung stromaufwärts des ersten Ventils angeschlossen ist,
- ein drittes Ventil (44) zur gesteuerten Unterbrechung der Zirkulation des Schmiermittels in der zweiten Auslassleitung,
• **dass** das Gehäuse (508) aus Metall ist,
• **dass** die Wand (510) an dem Gehäuse (508) der Zelle (506) angebracht ist und
• **dass** die Wand (510) aus Poly(ethylenterephthalat) hergestellt ist.

2. Anlage nach Anspruch 1, **dadurch gekennzeichnet, dass** die Zelle (506) einen Hohlraum (508C) zur Aufnahme der Wand (510) und eine Gewindescheibe (512) zur Fixierung der Wand in dem Hohlraum umfasst.

3. Anlage nach einem der Ansprüche 1 und 2, **dadurch gekennzeichnet, dass** der Sensor (50) ein Sensor zur Bestimmung des Eisengehalts der Probe des Schmiermittels ist.

4. Anlage nach einem der Ansprüche 1 bis 3, **dadurch gekennzeichnet, dass** das Gehäuse (508) aus einem Nichteisenmetall oder einer Nichteisenlegierung, insbesondere aus einer Legierung auf Aluminiumbasis, hergestellt ist.

5. Anlage nach einem der Ansprüche 1 bis 4, **dadurch gekennzeichnet, dass** die Röntgenstrahlenquelle (502) und der Röntgenstrahlendetektor (504) auf einer Abdeckung (514) angebracht sind, die sie in Bezug auf das Gehäuse (508) und die Wand (510) positioniert, und dass diese Abdeckung eine Abschirmung gegen die Ausbreitung der Röntgenstrahlen bildet.

6. Anlage nach einem der vorhergehenden Ansprüche, **dadurch gekennzeichnet, dass** eine Zielachse (A502) der Röntgenstrahlenquelle (502) und eine Zielachse (A504) des Röntgenstrahlendetektors (504) zwischen sich einen Winkel (α) zwischen 20 und 25°, vorzugsweise in der Größenordnung von 22°, bilden.

7. Anlage nach einem der vorhergehenden Ansprüche, **dadurch gekennzeichnet, dass** die Wand (510) eine Dicke von weniger als oder gleich 200 µm, vorzugsweise weniger als oder gleich 150 µm oder weniger, bevorzugt weiterhin in der Größenordnung von 125 µm, hat.

8. Anlage nach einem der vorhergehenden Ansprüche, **dadurch gekennzeichnet, dass** der Sensor (50) zur Bestimmung des Gehalts an einem vorbestimmten chemischen Element auf der zweiten Auslassleitung (42) angeordnet ist.

9. Anlage nach einem der vorhergehenden Ansprüche, **dadurch gekennzeichnet, dass** sie ferner einen Sensor (48) zur Bestimmung des Basizitätsindexes (BN) des Schmiermittels aufweist, der auf der zweiten Auslassleitung (42) angeordnet ist und mit dem der Basizitätsindex des Schmiermittels am Ausgang des Puffertanks (26) bestimmbar ist.

10. Automatisiertes Verfahren zur Verfolgung der Änderung des Gehalts an einem vorbestimmten chemischen Element eines Schmiermittels, das in einer Ausrüstung (M) zirkuliert, mittels einer Anlage (2) nach Anspruch 8, **dadurch gekennzeichnet, dass** es mindestens die folgenden Schritte umfasst:
a) Schließen des ersten Ventils (20),
b) Öffnen des zweiten Ventils (32) und Schließen des dritten Ventils (44), um den Puffertank aus einer in der Leitung (4) stromaufwärts des ersten Ventils gespeicherten Schmiermittelmenge (L; L') zu versorgen,
c) Öffnen des dritten Ventils (44), um das in dem Puffertank (26) vorhandene Schmiermittel durch die zweite Auslassleitung (42) bis in eine Zelle des Sensors (50) zirkulieren zu lassen,
d) Verwenden des Sensors (50), um den Gesamtgehalt an einem vorbestimmten chemischen Element des Schmiermittels am Ausgang des Puffertanks (26) zu bestimmen.

11. Verfahren zur Überwachung des Betriebs einer Ausrüstung (M), die sich an Bord eines Schiffes befindet, **dadurch gekennzeichnet, dass** es die Bestimmung des Gesamtgehalts eines vorbestimmten chemischen Elements, insbesondere Eisen, eines Schmiermittels, umfasst, das in der Ausrüstung zirkuliert, durch die Durchführung eines Verfahrens nach Anspruch 10.

12. Verfahren nach Anspruch 11 zur Bestimmung des Gesamteisengehalts eines Schmiermittels, das in einem Schiffsmotor (M) zirkuliert.

## Claims

1. An installation (2) for following up the evolution of the quality of a lubricant contained in a piece of equipment (M), this installation comprising:
- at least one conduit (4) for circulating (F1) the lubricant, this conduit being connected, upstream, to the piece of equipment and downstream, to a recovery pan (6),
- a sensor (50) for determining, by the X fluorescence technique, the overall content in a predetermined chemical element, of a lubricant sample, this sensor comprising an X-ray source (502), an X-ray detector (504) and a cell (506) intended to contain a lubricant sample to be analyzed and including a casing (508)
- a reservoir (26) for accumulating lubricant;
- said sensor (50) being configured to determine the overall content in a predetermined chemical element, of a lubricant sample, at the outlet of the reservoir;
- a first controlled interruption valve (20) for the circulation (F1) of the lubricant in the conduit (4);
- a first bypass line (28), connected on the one hand to the conduit and, on the other hand to the reservoir;
- a second controlled interruption valve (32) for the circulation of the lubricant in the first bypass line;
a second discharge line (42) for the lubricant, from the reservoir to the recovery pan; and
- a wall (510), which forms a window for letting through the X-rays from the source (502) or going towards the detector (504), made in polymer;
**characterized**
• **in that** the reservoir is a buffer reservoir (26) for accumulating lubricant,
• **in that** the installation includes
- the first bypass line (28) connected to the conduit upstream of the first valve,
- a third controlled interruption valve (44) for the circulation of the lubricant in the second discharge line,
• **in that** the casing (508) is in metal
• **in that** the wall (510) is in mounted on the casing (508) of the cell (506) and
• **in that** the wall (510) is made in polyethylene terephthalate).

2. The installation according to claim 1, **characterized in that** the cell (506) comprises a hollow housing (508C) for receiving the wall (510) and a threaded washer (512) for immobilization of the wall in the hollow housing.

3. The installation according to one of claims 1 and 2, **characterized in that** the sensor (50) is a sensor for determining the iron content of the lubricant sample.

4. The installation according to one of claims 1 to 3, **characterized in that** the casing (508) is made in a metal or in a non-ferrous alloy, notably in an alloy based on aluminium.

5. The installation according to one of claims 1 to 4, **characterized in that** the X-ray source (502) and the X-ray detector (504) are mounted on a lid (514) which positions them relatively to the housing (508) and to the wall (510) and **in that** this support forms a shield against the propagation of X-rays.

6. The installation according to one of the preceding claims, **characterized in that** a targeting axis (A502) of the X-ray source (502) and a targeting axis (A504) of the X-ray detector (504) form between them an angle (α) comprised between 20 and 25°, preferably of the order of 22°.

7. The installation according to one of the preceding claims, **characterized in that** the wall (510) has a thickness of less than or equal to 200 µm, preferably less than or equal to 150 µm, still preferably of the order of 125 µm.

8. The installation according to one of the preceding claims, **characterized in that** the sensor (50) for determining the content in a predetermined chemical element is positioned on the second discharge line (42).

9. The installation according to one of the preceding claims, **characterized in that** it further comprises a sensor (48) for determining the basicity index (BN) of the lubricant, positioned on the second discharge line (42) and which allows determination of the basicity index of the lubricant at the outlet of the buffer reservoir (26).

10. An automated method for following up the evolution of the content in a predetermined chemical element of a lubricant circulating in a piece of equipment (M), by means of an installation (2) according to claim 9, **characterized in that** it comprises at least steps consisting of:
a) closing the first valve (20),
b) opening the second valve (32) and closing the third valve (44) for supplying the buffer reservoir from an amount (L ; L') of lubricant accumulated in the conduit (4), upstream from the first valve,
c) opening the third valve (44) for having the lubricant present in the buffer reservoir (26) circulate through the second discharge line (42) as far as into a cell of the sensor (50).
d) using the sensor (50) for determining the overall content in a predetermined chemical element of the lubricant at the outlet of the buffer reservoir (26).

11. A method for following up the operation of a piece of equipment (M) loaded onboard a ship, **characterized in that** it comprises the determination, onboard the ship, of the overall content in a predetermined chemical element, notably in iron, of a lubricant circulating in the piece of equipment by applying a method according to claim 10.

12. The method according to claim 11 for determining the overall content in iron, of a lubricant circulating in a ship engine (M).
